# EUROPEAN PATENT APPLICATION

(11) **EP 2 471 497 A1**
(43) Date of publication of application: **04.07.2012**
(21) Application number: 10811880.3
(22) Date of filing: 25.08.2010
(51) Int. Cl.: A61F 2/82, A61L 29/00, A61L 31/00

(54) **MEDICAL DEVICE FOR PLACEMENT INTO A LUMEN AND MANUFACTURING METHOD THEREOF**

(30) Priority: 26.08.2009 JP 2009195350
(71) Applicant: Otsuka Medical Devices Co., Ltd., Tokyo 101-8535 (JP)
(72) Inventor: EGASHIRA, Kensuke, Fukuoka-shi Fukuoka 812-8581 (JP); TSUJIMOTO, Hiroyuki, Hirakata-shi Osaka 573-1132 (JP); HARA, kaori, Hirakata-shi Osaka 573-1132 (JP); TSUKADA, Yusuke, Hirakata-shi Osaka 573-1132 (JP); BANDO, Yohei, Hirakata-shi Osaka 573-1132 (JP); MANABE, Matsuya, Tokyo 151-0063 (JP)
(74) Representative: von Kreisler Selting Werner
(86) International application number: PCT/JP2010/064330
(87) International publication number: WO 2011/024831

(57) **Abstract**

The present invention provides a medical device for placement into a lumen such as a stent and a catheter whose surface is uniformly and sufficiently coated with a drug, and a process thereof with easy way and with low cost. The medical device is coated with mixed particles of drug particles whose surface is modified with positive-charge and biocompatible nanoparticles. In the invention, a drug can be taken into a cell through the dissolution of the drug particle together with the biocompatible nanoparticle after a DES is placed in a biological body.

## Description

### TECHNICAL FIELD

The present invention relates to a medical device for placement into a lumen such as a stent and a catheter which is placed at a stenosis or occluded part arising in an intravital lumen to keep the lumen open, which is coated with a drug and is a drug-dissolution type; and a method thereof.

### BACKGROUND ART

The current advancement of medicine brings about a remarkable development of the treatment/prevention of various diseases such as infection disease, but patients of arteriosclerotic disease or the like which is caused by bad lifestyle tends to increase. In particular, patients of arteriosclerotic disease such as myocardial infarction, angina, stroke, and peripheral vascular disease are increasing more and more in Japan, in connection with the westernization of lifestyle and the aging. As a method for surely treating such arteriosclerotic disease, percutaneous transluminal angioplasty (hereinafter, referred to as "PTA") is generally used, which is an angioplasty to surgically expand the stenosis or occluded part in blood vessel, for example, percutaneous transluminal coronary angioplasty in coronary artery which is typical.

The PTA is a technique for recovering the blood flow, in which a balloon catheter (a tube having a balloon at its tip) or a stent is inserted from an arm or femor artery, it is placed at a stenosis in the coronary artery, then the balloon attached at the tip is blown up to expand the stenotic blood vessel. This technique can expand an intravascular lumen in a lesion site to increase the blood flow in the intravascular lumen. The PTA is used for the treatment of arteriosclerotic disease as well as shunt stenosis which arises at an arm of a hemodialysis patient.

In general, the PTA-treated blood vessel is damaged such as detachment of endothelial cell and injury of elastic lamina, and the vascular intima grows because of the healing reaction in the vascular wall, thereby patients whose stenosis lesion site is opened by PTA can suffer from restenosis at a rate of about 30 to 40 %.

In more detail, the main cause of restenosis in human beings is thought to be the inflammatory process (adhesion/invasion of a monocyte) arising 1 to 3 days after the PTA, and the forming process of intimal thickening (smooth muscle cell) whose growth is the most about 45 days after the PTA. Once the restenosis comes up, it is necessary to do the PTA again. Accordingly, the establishment of the method for the prevention and treatment has been desired.

Then, it has been suggested to try reducing the rate of restenosis by releasing a drug topically for a long time at a site for placement in a lumen, using a drug-dissolution type of a medical device for placement into a lumen wherein an anti-inflammatory agent or an inhibitor of smooth-muscle cell proliferation is supported on the surface of stent or balloon catheter which is made of metal or polymer material. For example, Patent Reference 1 suggests a drug-eluting stent (hereinafter, abbreviated as "DES") wherein the body of a stent is coated with a biocompatible nanoparticle including a bioactive substance for the treatment, and a process thereof, which discloses a spherical crystallization technique as a process of a biocompatible nanoparticle.

However, a poorly water-soluble drug which is hardly dissolved in water such as probucol and cilostazol which have anti-thrombus activity is hard to be included in a biocompatible nanoparticle by spherical crystallization technique. Actually, when making probucol included in a particle by said spherical crystallization technique, the content of probucol in a PLGA nanoparticle was only about 0.5 %, which indicated that little drug was included. Thus, according to the method of Patent Reference 1, it was impossible to prepare a medical device for placement into a lumen wherein the surface is coated with a sufficient amount of a poorly water-soluble drug.

Patent Reference 2 discloses a method of attaching a drug on a carrier by dipping a stent or a catheter which is a carrier to a solution of a drug which is water-insoluble and drying the carrier. In the method disclosed in Patent Reference 2, however, the attached amount was limited, accordingly it was difficult to attach a sufficient amount of a drug on a carrier. In addition, the attached drug was released in a short time, thereby it was also difficult to control the releasing time.

Patent Reference 3 discloses a drug-releasing control type of multilayered stent whose surface is coated with a drug ingredient and a biocompatible polymer as a second coating layer, wherein probucol is listed as one of the exemplified active ingredients. Patent Reference 4 discloses a medical device coated with a biocompatible substance comprising a medicinal ingredient, and Patent Reference 5 discloses a drug delivery system that uses a balloon and an implant prosthesis (stent) which are at least partially coated with a coating agent comprising a drug and a carrier. Further, both of Patent References 4 and 5 disclose probucol as a drug.

However, the methods disclosed in Patent References 3 to 5 had a problem that it takes excessive time to elute a drug and then it is difficult to obtain an enough efficacy of a drug, because a drug ingredient is released in tandem with the decomposition of the biocompatible polymer layer. These methods need to use a solvent which can dissolve both of a drug ingredient and a biocompatible polymer in preparing a liquid coating agent of the drug ingredient and the biocompatible polymer. However, it was restrictive to find such solvent from the viewpoint of the combination of a biocompatible polymer and a drug. Consequently, the methods had a problem of lacking versatility as a coating technique.

In addition, using cilostazol which is another poorly water-soluble drug and has platelet aggregation inhibition action and so on, the same application of the drug to a medical device has been tried, there has been the same problem in the trials (Patent References 3, 6 to 19).

[Patent Reference 1] JP 2007-215620 A
[Patent Reference 2] JP 2005-538812 T
[Patent Reference 3] JP 2006-198390 A
[Patent Reference 4] JP 2007-528275 T
[Patent Reference 5] JP 2007-529285 T
[Patent Reference 6] JP 2007-117742 A
[Patent Reference 7] JP 2003-2900360 A
[Patent Reference 8] JP 2001-190687 A
[Patent Reference 9] JP 4473390 B
[Patent Reference 10] JP 2010-506837 T
[Patent Reference 11] JP 2010-506849 T
[Patent Reference 12] JP 2009-511195 T
[Patent Reference 13] JP 2009-511205 T
[Patent Reference 14] JP 2008-533044 T
[Patent Reference 15] JP 2008-505126 T
[Patent Reference 16] JP 2006-526652 T
[Patent Reference 17] JP 2005-531391 T
[Patent Reference 18] JP 2005-508671 T
[Patent Reference 19] JP 2004-523275 T

### SUMMARY OF INVENTION

### (Problem to be solved by the invention)

In consideration of the above problem, the object of the present invention is to provide a medical device for placement into a lumen such as a stent and a catheter, whose surface is uniformly and sufficiently coated with a poorly water-soluble drug and wherein the drug has a certain sustained-release property and can be sufficiently dissolved in a necessary period, and a method thereof.

### (Means to solve the problem)

The present inventors have extensively studied in order to solve the above-mentioned problems, and have succeeded in uniformly and sufficiently coating the body of a device with a drug which is difficult to be supported on a biocompatible nanoparticle, by making the surface of a poorly water-soluble drug positive-charged to provide a drug particle and then coating the body of the device with the drug particle together with a biocompatible nanoparticle, without embedding the poorly water-soluble drug in the biocompatible nanoparticle. Based upon the new findings, the present invention has been completed.

The first embodiment of the present invention to achieve the above purpose is a medical device for placement into a lumen whose body is coated with mixed particles of poorly water-soluble drug particles whose surface is modified with positive-charge and biocompatible nanoparticles. Wherein, the drug particles may be prepared in two or more kinds of drug particles using two or more kinds of drugs.

The second embodiment of the present invention is the above medical device for placement into a lumen wherein the surface of the drug particles is modified with positive-charge by attaching a cationic polymer to their surface. Wherein, the cationic polymer is preferably chitosan or a chitosan derivative. In addition, the cationic polymer is attached together with a water-soluble polymer such as polyvinyl alcohol to the surface of the drug particles.

The third embodiment of the present invention is the above medical device for placement into a lumen wherein the drug particles have a mean particle size of 0.1 µm to 5 µm.
The alternative third embodiment of the present invention is the above medical device for placement into a lumen wherein the biocompatible nanoparticles have a mean particle size of 1,000 nm or less, preferably 300 nm or less.

The fourth embodiment of the present invention is the above medical device for placement into a lumen wherein the biocompatible nanoparticle supports the same or different drug inside and/or on its surface besides the drug particle.

The fifth embodiment of the present invention is the above medical device for placement into a lumen wherein the biocompatible nanoparticle is composed of any one of polylactic acid, polyglycolic acid, poly(lactic-co-glycolic acid), and lactate-aspartate copolymer. Wherein, the biocompatible nanoparticle may be composed of a single compound or a mixture of two or more compounds selected from the above compounds.

The sixth embodiment of the present invention is the above medical device for placement into a lumen which is a drug-dissolution type stent whose body is coated with the drug particles and the biocompatible nanoparticles.

The seventh embodiment of the present invention is the above medical device for placement into a lumen which is a drug-dissolution type catheter whose surface is coated with the drug particles and the biocompatible nanoparticles, in particular an extensible drug-dissolution type catheter equipped with an extensible attachment whose surface is coated with the drug particles and the biocompatible nanoparticles. Wherein, the catheter with an extensible attachment includes a balloon catheter.

The eighth embodiment of the present invention is a liquid coating agent for coating the above medical device for placement into a lumen. Wherein, the said liquid coating agent is a suspension prepared by dispersing drug particles modified with positive-charge by attaching a cationic polymer to their surface and biocompatible nanoparticles in a water solution. Wherein, the drug particles modified with positive-charge by attaching a cationic polymer to their surface may be modified by attaching a cationic polymer beforehand in a step forming the drug particles, and/or the biocompatible nanoparticles may be also adhered with a cationic polymer by adding a cationic polymer in dispersing the drug particles and the biocompatible nanoparticles. Wherein, the drug particles modified with positive-charge by attaching a cationic polymer, the biocompatible polymers, and the cationic polymer mean the same as defined in the above medical device for placement into a lumen.

The ninth embodiment of the present invention is a process for preparing a medical device for placement into a lumen which comprises
(1) a step forming a drug particle by adding a solution of a drug in an organic solvent to an aqueous solution of a water-soluble polymer to provide a drug particle,
(2) a step forming a nanoparticle by adding a solution of a biocompatible polymer in an organic solvent to an aqueous solution of a water-soluble polymer to provide a biocompatible nanoparticle,
(3) a step preparing a liquid coating agent by dispersing the above-formed drug particle and the above-formed biocompatible nanoparticle to provide a liquid coating agent,
(4) a step modifying the surface of the drug particle with positive-charge by dissolving a cationic polymer in the aqueous solution in step (1) and/or the liquid coating agent in step (3), and
(5) a step coating the device with the above-prepared liquid coating agent by contacting the liquid coating agent and the body of the device to form a particle layer in a state of mixed particles of the drug particles and the biocompatible nanoparticles.

The tenth embodiment of the present invention is the above process wherein in step (4) the surface of the drug particle is modified with positive-charge by dissolving a cationic polymer in both the aqueous solution in step (1) and the liquid coating agent in step (3).

The eleventh embodiment of the present invention is the above process which further comprises a step milling the drug particles after step (1).

The twelfth embodiment of the present invention is the process wherein in step (1) the water-soluble polymer is polyvinyl alcohol and the cationic polymer is chitosan.

The thirteenth embodiment of the present invention is the above process wherein step (5) is carried out by electrophoresis, ultrasonic mist method, spray method, air brush method, wiping method or dipping method.

The fourteenth embodiment of the present invention is the process wherein the drug particles are composed of two or more kinds of drug particles which are in a layered state or in a mosaic state.
In addition, the present invention also includes each medical device for placement into a lumen prepared in the above ninth to fourteenth embodiments.

The fifteenth embodiment of the present invention is the above medical device for placement into a lumen, liquid coating agent, or process wherein the drug of the drug particle is cilostazol.

The sixteenth embodiment of the present invention is the medical device for placement into a lumen which is for preventing and/or treating thrombus, stenosis, and/or restenosis. In particular, the effect of the present invention can be exerted when a stent, a catheter, a balloon catheter or the like is used as a medical device, and a preferred drug in the drug particle is a drug having platelet aggregation inhibition action such as cilostazol.
In addition, the embodiment of the present invention also includes a method for preventing and/or treating thrombus, stenosis, and/or restenosis which comprises using the medical device for placement into a lumen, or use of the medical device for placement into a lumen for preventing and/or treating thrombus, stenosis, and/or restenosis.
Furthermore, the embodiment of the present invention also includes the above liquid coating agent for preventing and/or treating thrombus, stenosis, and/or restenosis. In this case, the liquid coating agent can be used in a body directly or through a medium other than the medical device for placement into a lumen.

### (Effect of the Invention)

According to the first embodiment, it is possible to coat the body of a device with mixed particles of poorly water-soluble drug particles whose surface is modified with positive-charge and biocompatible nanoparticles, thereby it is possible to provide a drug-dissolution type medical device for placement into a lumen whose body is uniformly and sufficiently coated with a drug which has been difficult to be supported on a biocompatible nanoparticle before. The surface of particle prepared by the current spherical crystallization technique generally has negative zeta potential, while the intravital cell wall is also negative-charged, thus the current technique had a problem that the adhesiveness of a particle to a cell was low due to electric repulsion. In the present invention, the surface of a drug particle is positive-charged, thus the cell-adhesiveness of a particle to a negative-charged cell wall is high and thereby the efficiency making a drug come in a cell can increase. In addition, the body of a device is coated with each of a drug particle and a biocompatible nanoparticle directly, thus the present invention can exert a sufficient drug activity, compared with the case that a drug is included in a biocompatible polymer layer, because it does not take so much time to make a drug dissolved.

According to the second embodiment, it is possible to easily positive-charge the surface of the drug particle in the medical device for placement into a lumen of the first embodiment by modifying the surface of the drug particle with positive-charge using a cationic polymer.

According to the third embodiment, it is possible to increase the intravital incorporation of the drug particle in the medical device for placement into a lumen of the first or second embodiment by making the mean particle size of the drug particle in the range of 0.1 µm to 5 µm. It is possible to increase the stacking character of a drug on the surface of a device and the permeability effect of a drug into a target cell, when a biocompatible nanoparticle has a mean particle size of less than 1,000 nm, preferably 300 nm or less. Thanks to such limitation of the size, it is possible to make a drug-dissolution type of a medical device for placement into a lumen wherein the drug particle is uniformly layered. In the present invention, the mean particle size of a drug particle means a mean particle size derived by laser diffraction-scattering mentioned below, and the mean particle size of a biocompatible nanoparticle means a mean particle size derived by dynamic light scattering.

According to the fourth embodiment, it is possible to increase the amount of a drug supported on the body of a device in the medical device for placement into a lumen of any one of the first to third embodiments by supporting the same or different drug inside the biocompatible nanoparticle and/or on its surface besides the drug particle. In addition, the present invention is a medical device for placement into a lumen which exhibits both a rapidly-releasing property via a drug particle from the surface of a device and a gradually releasing property from the inside or surface of a biocompatible nanoparticle, after the medical device is placed in a target site. And, if using a drug different from the drug in the drug particle, for example, plural drugs whose efficacies and action mechanisms are different; some drug potencies can be enhanced because of a synergistic effect with each ingredient. In the specification, it can be called "include" to support a drug in a biocompatible nanoparticle.

According to the fifth embodiment, it is possible to provide the medical device for placement into a lumen of any one of the first to fourth embodiments wherein the biocompatible nanoparticle is composed of any one of polylactic acid (PLA), polyglycolic acid (PGA), poly(lactic-co-glycolic acid) (PLGA), or lactate-aspartate copolymer (PAL), which has low irritant and toxic properties for biologic bodies and can release a drug gradually due to the decomposition of the biocompatible polymer in case that a drug is included in the particle.

According to the sixth embodiment, it is possible to provide the medical device for placement into a lumen of any one of the first to fifth embodiments wherein the drug particle and the biocompatible nanoparticle are coated on the body of a stent, thereby it is possible to release the drug in the placement of a lumen for a long time and locally to effectively decrease restenosis.

According to the seventh embodiment, it is possible to coat a drug particle and a biocompatible nanoparticle on the surface of the medical device for placement into a lumen of the first to fifth embodiments, specifically the surface of the extensible part of a catheter equipped with the extensible part, thereby, after inserting the catheter into a stenosis part in blood vessel, and extending the extensible part to expand the stenosis part, it is possible to prepare an extensible drug-dissolution type catheter which can topically release a drug from the extensible part to reduce the restenosis effectively.

According to the eighth embodiment, it is possible to provide a liquid coating agent for coating the above medical device for placement into a lumen, thereby it is possible to coat various medical devices for intravital placement such as the above-mentioned stent and catheter.

According to the ninth embodiment, it is possible to coat the body of a device with mixed particles of a drug particle having high cellular adhesiveness whose surface is positive-charged and a biocompatible nanoparticle to form a uniform particle layer on the body of a device, thereby it is possible to prepare a drug-dissolution type of a medical device for placement into a lumen which can make the drug particle efficiently delivered in a cell and is easy to handle, by easy way and with low cost. In addition, it is not necessary to use a solvent which can dissolve both of a biocompatible polymer and a drug, thereby the process of the invention is a versatile preparing-method which does not depend to the combination of a drug and a biocompatible polymer.

According to the tenth embodiment, in the process for preparing a medical device for placement into a lumen of the ninth embodiment, it is possible to drastically depress the aggregation of drug particles in the step forming a drug particle by dissolving a cationic polymer in both the aqueous solution in the step forming a drug particle and the liquid coating agent and also it is possible to enough modify the surface of a drug particle with positive-charge. In addition, the surface of the biocompatible nanoparticle is also positive-charged in this embodiment, hence, in case that the body of a device is conductive, it is possible to form a particle layer uniformly on the body of a device having a complicated shape by contact a liquid coating agent with the body of a device which is electrified in the process of attaching particle. In addition, it is possible to control the thickness of a particle layer by controlling the electric voltage or the time of the electrification.

According to the eleventh embodiment, in the process for preparing a medical device for placement into a lumen of the ninth to tenth embodiments, it is possible to increase the biologic uptake of the drug by comprising a step micro-milling the drug particle after the step forming the drug particle. In addition, it is possible to form a more uniform particle layer on the body of a device, and increase the amount of the drug particle attached on the body of a device. The process of milling a drug particle can be carried out in the process for preparing the medical device for placement into a lumen and the liquid coating agent of the first to eighth embodiments, thus the same effect is expected therein.

According to the twelfth embodiment, in the process for preparing a medical device for placement into a lumen of any one of the ninth to eleventh embodiments, it is possible to make the dispersibility of the drug particle to water higher, drastically depress the agglutination of drug particle and then increase the yield ratio in the step forming a drug particle, by dissolving polyvinyl alcohol and chitosan as a water-soluble polymer in the step forming a drug particle.

According to the thirteenth embodiment, in the process for preparing a medical device for placement into a lumen of any one of the ninth to twelfth embodiments, it is possible to efficiently and easily form a uniform particle layer by carrying out the process of attaching particles through any one of electrophoresis, ultrasonic mist method, spray method, air brush method, wiping method and dipping method.

According to the fourteenth embodiment, in the process for preparing a medical device for placement into a lumen of any one of the ninth to thirteenth embodiments, it is possible to prepare a medical device for placement into a lumen which can systematically control each dissolution time of two or more kinds of drugs by forming a particle layer comprising different types of drug particles in a layered state or in a mosaic state, and by attaching a drug particle which should be released in a short time after placing a device in a biologic body in the outer layer, and attaching a drug particle which should be released after a given time in the inner layer.

According to the fifteenth embodiment, it is possible to provide the above-mentioned medical device for placement into a lumen in which cilostazol is used as a drug of the drug particle, for example, which is expected to prevent restenosis in using it in a stent through its action for inhibiting smooth muscle cell proliferation, platelet aggregation inhibition action, anti-inflammatory action and so on, and additionally the other various actions of cilostazol are also expected to be exerted.

According to the sixteenth embodiment, it is expected to improve functional disorders of the applied organ by using the above-mentioned medical device for placement into a lumen. It is expected to improve, specifically, heart failure, myocardial infarction, cerebral infarction, chronic kidney disease (CKD), and so on.

### BRIEF DESCRIPTION OF DRAWINGS

Fig 1. illustrates a frame format of the structure of a drug particle used for the medical device for placement into a lumen, whose particle surface is positive-charged.
Fig 2. illustrates a frame format of the structure of a biocompatible nanoparticle used for the medical device for placement into a lumen, whose particle surface is positive-charged.
Fig. 3 shows a diagrammatic illustration of the electrophoresis apparatus used for preparing a DES in the present invention.
Fig. 4 is a cross-sectional frame format which shows a state in which a particle layer is formed on a metallic fiber as the body of a stent.
Fig. 5 is a cross-sectional frame format which shows a state in which a particle layer and a biodegradable polymer layer are formed on a metallic fiber as the body of a stent.
Fig. 6 is a cross-sectional frame format which shows a state in which a negative-charged resin layer and a particle layer are layered on the balloon part of a catheter.
Fig. 7 shows the transitional result of the minimum vessel diameter in Test 3.
Fig. 8 shows the result in the control group among HE-stained pathological specimens of the cross-sectional vessel which was extirpated 28 days after the placement of the stent in Test 3.
Fig. 9 shows the result in the cilostazol-attached stent group among HE-stained pathological specimens of the cross-sectional vessel which was extirpated 28 days after the placement of the stent in Test 3.

### EXPLANATIONS OF LETTERS OR NUMERALS

- 1:: drug particle
- 2:: biocompatible nanoparticle
- 3:: polyvinyl alcohol
- 4:: cationic polymer
- 5:: electrophoresis apparatus
- 6:: bath
- 7:: liquid coating agent
- 8:: stent body
- 9:: positive electrode
- 10:: metallic fiber
- 11:: particle layer
- 12:: biodegradable polymer layer
- 13:: balloon part
- 15:: negative-charged resin layer

### DESCRIPTION OF EMBODIMENTS

Hereinafter, the embodiments of the present invention are in detail illustrated, referring to the drawings. The process for preparing a medical device for placement into a lumen of the present invention comprises
(1) a step forming a drug particle to provide a drug particle, (2) a step milling the drug particle, (3) a step forming a nanoparticle to provide a biocompatible nanoparticle, (4) a step preparing a liquid coating agent by dispersing the above-formed drug particle and the above-formed biocompatible nanoparticle to provide a liquid coating agent, (5) a step modifying the surface of the drug particle with positive-charge, and (6) a step coating the device with the above-prepared liquid coating agent by coating the body of the device with the drug particle and the biocompatible nanoparticle to form a particle layer in a state of mixed particles of the drug particle and the biocompatible nanoparticle. Hereinafter, each process is explained from step forming drug particle to process of attaching particle, sequentially.

### (1) Step forming drug particle

The drug particle used herein can be prepared by crystallization method.
In the crystallization method, two kinds of solvents, a good solvent in which the poorly water-soluble drug used in the present invention is soluble and a poor solvent in which the drug is insoluble on the contrary, are used. As the good solvent, an organic solvent which dissolves the drug and are well mixed with a poor solvent such as acetone is used herein. As the poor solvent, an aqueous solution of polyvinyl alcohol or the like is generally used.

In the operating process, first of all, the drug is dissolved in a good solvent. The solution of the drug is added dropwise in a poor solvent with stirring. When the interaction (inter-solubility) between the good solvent and the drug is stronger than that of the good solvent and the poor solvent, an emulsion dropping arises in the mixture. On the contrary, when the interaction between the good solvent and the drug is weaker than that of the good solvent and the poor solvent, a crystal of the drug arises in the mixture and thereby a suspension is produced.

The drug used in preparing the drug particle is not limited, but a poorly water-soluble drug which has been hard to be included in a biocompatible nanoparticle or coated on the surface of a device before can be used herein. The poorly water-soluble drug used herein is not clearly defined, but for example, it can be defined as the solubility of the drug in water is that "very slightly soluble; the volume of solvent required for dissolving 1 g or 1 mL of solute is 1,000 mL or more" defined in the Japanese Pharmacopoeia XXV.
Some examples of the poorly water-soluble drug include, but are not limited to, cilostazol, probucol, simvastatin, tacrolimus, sirolimus, zotarolimus, everolimus, and paclitaxel.

A specific solvent among the good solvents used in the above crystallization method is selected depending on the drug used therein, but it is necessary to select a solvent which is safe for a human body (because the drug particle is used as a material of medical device which is placed in a human body) and little harmful to the environment. For example, an organic solvent classified as Class 3 solvents defined in "Impurities: Guideline for Residual Solvents" issued by Pharmaceutical and Medical Safety Bureau of the Health, Labor and Welfare Ministry is preferable. The specific solvent classified therein includes acetone, ethanol, 1-butanol, ethyl acetate, diethyl ether, and tetrahydrofuran which are organic solvents having a low melting point, preferably acetone which has little adverse affect for the environment and a human body, or a mixture of acetone and ethanol.

Like the case of the good solvent, a specific solvent among the poor solvents is also selected depending on the drug used therein, for example, it includes water or an aqueous solution of a surfactant. A preferred example thereof is an aqueous solution of a water-soluble polymer as a surfactant such as polyvinyl alcohol. The water-soluble polymer used herein other than polyvinyl alcohol includes polyethylene glycol, polyvinylpyrrolidone, hydroxymethylcellulose, and hydroxypropylcellulose.

The concentration of polyvinyl alcohol in the aqueous solution, the mixture ratio of acetone and ethanol, and the condition of the crystallization are not specifically limited, but may be decided suitably depending on the drug of the drug particle, the particle size of the crystal, etc. The higher the concentration of polyvinyl alcohol in the aqueous solution is, the better the adhesiveness of polyvinyl alcohol to the particle surface is, and thereby the re-dispersibility of the drug to water after drying it. However, when the concentration of polyvinyl alcohol in the aqueous solution is beyond a certain level, the viscosity of the poor solvent is enhanced so that the diffuseness of the good solvent can be affected negatively.
Accordingly, the concentration of polyvinyl alcohol in the aqueous solution is preferably 0.1 % (w/w) to 10 % (w/w), more preferably about 2 % (w/w) when the organic solvent is removed after preparing the drug particle and furthermore an excess of polyvinyl alcohol is removed, but it depends on the polymerization degree and saponification degree of the polyvinyl alcohol. And, when the organic solvent is removed from the suspension after forming the particle and then the residue is directly used in the process of attaching particle via the milling process, the concentration of polyvinyl alcohol in the aqueous solution is preferably 0.5 % (w/) or less, more preferably about 0.1 % (w/w).

Each amount of said good solvent and poor solvent used herein includes, but depends on the kinds of the used drug and solvent, for example, 10 to 1000 mL, preferably 20 to 500 mL of the good solvent, and 20 to 2000 mL, preferably 50 to 1000 mL of the poor solvent, per 1 g of the poorly water-soluble drug.

In general, many of the particles dispersed in a liquid are charged positively or negatively, and ions having opposite charge thereto can strongly gravitate to the particle surface to form a fixed layer. In addition, there is a diffuse layer outside the fixed layer, and then so called "diffuse electric double layers" are formed. Thus, it is guessed that a partial inside of the diffuse layer and the fixed layer move together with the particle.
The zeta potential means an electric potential on the glide plane arising by the above movement, which is based on the electric potential on the electrically neutral range enough depart from the particle. The higher the absolute value of the zeta potential becomes, the stronger the repulsion between the particles becomes, and thereby the stability of the particles gets to be enhanced. On the contrary, when the zeta potential makes an approach to zero, the particles can be easily agglutinated. Accordingly, the zeta potential can be used as an indicator of the dispersing state of particles.
Thus, using an aqueous solution of a water-soluble polymer in the process forming drug particles as a poor solvent, the surface of the drug particle can be coated with the water-soluble polymer, and thereby the zeta potential of the drug particle becomes higher and the dispersibility to water becomes higher, then the agglutination of the drug particle in the milling process mentioned below is suppressed. In particular, when a cationic polymer such as chitosan is used as a water-soluble polymer together with polyvinyl alcohol, the surface of the formed drug particle is modified with positive-charge with the cationic polymer to have a positive zeta potential, thereby the agglutination can be remarkedly suppressed.

### (2) Milling process

The drug particle prepared by crystallization method often has a particle size of a broad range of a hundred nm to several hundred µm, unless the drug is an amorphia. When the drug particle is layered on the body of a device without any treatment, the biologic uptake of the drug is low, and it is impossible to coat the body of a device with an appropriate amount of the drug because the drug particle cannot be uniformly layered. To overcome such trouble, a physically milling process can be set so that the mean particle size of the drug particle can be suitably in a given range.

When the mean particle size of the drug particle is adjusted to 5 µm or less, it becomes possible that the drug particle is more uniformly layered on the body of a device together with a biocompatible nanoparticle in the process of attaching particle mentioned below, compared with an unmilled drug particle. And, when the drug particle of the present invention is used in a stent or a catheter and the device is placed in a biologic body, the affinity between the drug and a target cell is enhanced and the biologic uptake of the drug particle is also enhanced. On the other hand, when the mean particle size is 0.1 µm or less, it is difficult to maintain the dispersibility of the drug particle as a single particle, then the drug particle is apt to be re-agglomerated quickly to lose the effect of the milled drug particle. Accordingly, the mean particle size of the milled drug particle is preferably in a range of 0.1 µm to 5 µm.

The method of mill used herein is preferably to mill a drug particle in a suspension state with a wet miller such as Disperse Mill (HOSOKAWA MICRON CORPORATION), Aquamizer (HOSOKAWA MICRON CORPORATION), MSC mill (Mitsui Kozan), and desktop-type ball mill (IRIE SHOKAI).

### (3) Step forming biocompatible nanoparticle

The biocompatible nanoparticle of the present invention can be prepared by emulsion solvent diffusion method (ESD method) which is a kind of crystallization methods to prepare a drug particle. The ESD method is a method which can control the preparation and growth of a crystal in a final process of a compound synthesis to design a spherical crystalline particle and directly prepare it while controlling its physicality. The spherical crystallization technique can easily form a uniform particle without considering a problem such as the residue of a catalyst or a material compound, because it can form a microparticle via a physical-chemical approach and the obtained particle is a substantially spherical particle.

In the ESD method, two kinds of solvents are used, i.e. a good solvent which can dissolve a biocompatible polymer and a poor solvent which adversely cannot dissolve a biocompatible polymer. The good solvent used herein which can dissolve a biocompatible polymer is an organic solvent which can be well mixed with a poor solvent such as acetone. And the poor solvent used herein is generally an aqueous solution of polyvinyl alcohol or the like.

When a solution obtained by dissolving a biocompatible polymer in a good solvent is added dropwise with stirring into a poor solvent which cannot dissolve the biocompatible polymer, the good solvent in the mixture comes to diffuse rapidly into a poor solvent. Then, the good solvent is self-emulsify to form an emulsion dropping of the submicron good solvent. Furthermore, the interdiffusion between a good solvent and a poor solvent can make the solubility of the biocompatible polymer in the emulsion dropping lowed, then to form a crystalline particle of the biocompatible polymer having a mean particle size of 1,000 nm or less.

The ESD method can form a nanoparticle via a physical-chemical approach wherein the nanoparticle is a substantially spherical particle, the method makes it possible to easily form a uniform particle without considering a problem such as the residue of a catalyst or a material compound. Then, the organic solvent which is a good solvent is removed in vacuo and the residue is dried to give a nanoparticle powder. The types of a good solvent and a poor solvent, the concentration of an aqueous solution of polyvinyl alcohol as a poor solvent, etc. are as defined above.

The biocompatible polymer used herein is preferably a biocompatible polymer having low irritant/toxic properties for biologic bodies which can be metabolized in vivo after administered. And preferably, when the nanoparticle includes a drug, the included drug is gradually released. The material used herein for such purpose is preferably PLGA (copoly lactic acid/glycolic acid).
The molecular weight of PLGA is preferably a range of 5,000 to 200,000, more preferably a range of 15,000 to 25,000. The composition ratio of lactic acid and glycolic acid is 1:99 to 99:1, preferably lactic acid/glycolic acid is 1:0.333.
The biocompatible polymer besides PLGA includes a chemically-synthesized biocompatible polymer such as polyglycolic acid, polylactic acid, polymalic acid, polyhydroxy acid, polyhydroxybutyric acid, polybutylene succinate, and polycaprolactam. These copolymers can be used directly or after modified with a charge group such as an amino acid or functionalized. The biocompatible polymers besides the above-mentioned ones include naturally-occurring acetylcellulose, chitin, chitosan, gelatin, and collagen, as well as microbially-derived polyhydroxybutyrate and the like.

Then, the obtained suspension of nanoparticle is used in the next step preparing a liquid coating agent, without any treatment, or after removing the organic solvent as a good solvent if necessary and then powdering it once by lyophilization if necessary. Preferably, if the nanoparticle is used in the next step as a suspension, the lyophilization becomes unnecessary to simplify the process.

The main purpose of the biocompatible nanoparticle used herein is to increase the coating amount of the drug particle by layering the nanoparticle on the body of a device in a state of mixing with the drug particle. Accordingly, in the above step forming a nanoparticle, the process of the biocompatible nanoparticle comprising only a biocompatible polymer is explained. In addition, it is possible to prepare a nanoparticle wherein a drug is supported inside or on its surface by dissolving a drug together with a biocompatible polymer in a good solvent. When using such nanoparticle, it is possible to make the total amount of a drug coated on the body of a device increased and also make the therapeutic effect enhanced through the different efficacies and action mechanisms of the drugs supported in the drug particle and the nanoparticle, and the different dissolution rates thereof.

The drug supported in a biocompatible nanoparticle can be a drug whose efficacy and action mechanism is the same as that of the drug in the drug particle or different from that thereof. The ingredient having a different efficacy or action mechanism includes, but not limited thereto, for example, an antiplatelet drug such as aspirin, dipyridamole, heparin, antithrombin drug, and fish oil; an inhibitor of smooth-muscle proliferation such as low-molecular-weight heparin, and angiotensin-converting enzyme inhibitor; an anticancer agent such as vincristine sulfate, vinblastine sulfate, vindesine sulfate, irinotecan hydrochloride, paclitaxel, docetaxel hydrate, methotrexate, and cyclophosphamide; antibiotics such as mitomycin C; an immunosuppressive agent such as sirolimus, and tacrolimus hydrate; an anti-inflammatory agent such as a steroid; a lipid-regulating agent such as cerivastatin sodium, and lovastatin; a gene compound such as plasmid DNA, gene, siRNA, decoy nucleic acid medicine (decoy), polynucleotide, oligonucleotide, antisense oligonucleotide, ribozyme, aptamer, interleukin, and intercellular messenger (cytokine); and a receptor tyrosine kinase inhibitor such as imatinib and PTK787. The biocompatible nanoparticle can support one ingredient among the above drugs, or two or more drugs having different efficacies and action mechanisms to try to make some synergistic effect based on the different drugs.
The amount of the drug supported on the biocompatible nanoparticle depends on the type of drug, but, it is, for example, 20 % (w/w) or less.

In case that the drug has a relatively high solubility for water and an extremely low solubility for an organic solvent, the drug can be dissolved in a poor solvent beforehand, then a solution of a biocompatible polymer in a good solvent can be added dropwise thereto. According to this method, it is possible to prepare a nanoparticle having a water-soluble drug in a high concentration because the drug can be supported on the particle surface as well as partially in a matrix of the biocompatible polymer. The above-mentioned poor solvent or good solvent means a poor solvent or a good solvent for a biocompatible polymer.

In preparing a nanoparticle including an anionic drug which exists as an anionic molecular in water, the water-soluble anionic drug which is dispersed/mixed in a good solvent is supposed to leak out and solve in a poor solvent, and only a polymer for forming a nanoparticle can precipitated, thus as a result the nanoparticle can include few anionic drug. In such case, by adding a cationic polymer such as chitosan in a poor solvent, the cationic polymer adsorbed on the nanoparticle surface can interact with the anionic drug on the surface of the emulsion dropping to prevent the leakage of the anionic drug to a poor solvent, thereby the included rate of an anionic drug inside a nanoparticle can be enhanced. The above-mentioned poor solvent or good solvent means a poor solvent or a good solvent for a biocompatible polymer.

The biocompatible nanoparticle used herein is not limited as long as the mean particle size thereof is less than 1,000 nm, but the biocompatible nanoparticle is necessary to be uniformly layered together with a drug particle on the surface of a device such as a stent and a catheter. The nanoparticle supporting a drug needs to be incorporated in a cell to provide the drug in a stenosis site wherein a stent or catheter is placed. Thus, in order to enhance the layering property on the surface of a device and the penetrating effect of a drug into a target cell, the mean particle size can be made to preferably 300 nm or less so that the nanoparticle delivered to the target site can be easily taken into a cell by plasmalemmal endocytosis.

### (4) Step preparing liquid coating agent

The drug particle and biocompatible nanoparticle prepared as above is dispersed in water to a suspension thereof to prepare a liquid coating agent for using in the process of attaching particle mentioned below.

The mixture ratio of the drug particle/biocompatible nanoparticle in the liquid coating agent is 10:90 to 90:10 by weight, preferably 15:85 to 85:15 by weight, which can be decided based on the type of drug particle, particle size, the amount of drug particle coated on the body of a device, or the releasing rate. For example, when raising the amount of biocompatible nanoparticle in the mixture, it is supposed that the amount of the coated drug particle is increased and the releasing rate of the drug particle from the body of a device is delayed. On the contrary, when raising the amount of drug particle, it is supposed that it comes out the opposite result. In case that cilostazol is used herein, the mixture ratio of the drug particle/biocompatible nanoparticle may be defined as above, preferably 50:50 to 15:85, in particular, the ratio of 1:2 can bring in a good effect.

### (5) Step of modification with positive-charge

The surface of drug particle is modified with positive-charge by being coated with a cationic polymer beforehand. The surface of particle prepared by a conventional spherical crystallization technique has a negative zeta potential in general, thereby the conventional technique has a problem that the cellular adhesiveness of the particle is worsened due to electric repelling force because the intravital cell wall is also negative-charged. Hence, when the surface of drug particle is electrically charged by using a cationic polymer to have positive zeta potential, the adhesiveness of drug particle to a negative-charged cell wall can be increased and then the transitivity of a drug into a cell can be enhanced.

The cationic polymer used herein includes chitosan and chitosan derivative, cationized cellulose, a polyamino compound (such as polyethylenimine, polyvinylamine, and polyallylamine), polyamino acid (such as polyornithine, polylysine, and polyarginine), polyvinylimidazole, polyvinylpyridinium chloride, alkylaminomethacrylate quaternary salt polymer (DAM), and alkylaminomethacrylate quaternary salt·acrylamide copolymer (DAA), preferably chitosan or a derivative thereof.

The chitosan is a natural cationic polymer having many glucosamines that is a kind of a sugar having an amino group, which is contained in an outer envelope of shrimps and crabs. The chitosan is broadly used as a material for cosmetics, foods, clothes, pharmaceuticals, etc. because it has some properties such as emulsion stability, shape retention, biodegradability, biocompatibility, and antibacterial activity. The chitosan can be added to a solution of a drug in a poor solvent for the drug to prepare a drug particle which has unharmful effect to human body and high safity.

When using a more strongly cationic polymer, the zeta potential becomes a more stronger positive-charge, then the electrical adsorbability in the step attaching particle mentioned below is enhanced and the repelling force between particles is enhanced to stabilize particles in a suspension. For example, a partial site of a cationic chitosan can be quaternized to prepare a chitosan derivative (cationic chitosan) such as N-[2-hydroxy-3-(trimethylammonio)propyl]chitosan chloride, which is preferable for use in the present invention.

In order to modify with positive-charge in the present step, it can be achieved by dissolving a cationic polymer in an aqueous solution in the step forming drug particle mentioned above, or adding a cationic polymer in a liquid coating agent prepared in the step preparing liquid coating agent. In particular, when a cationic polymer is dissolved in an aqueous solution in the step forming drug particle and furthermore a cationic polymer is also dissolved in a liquid coating agent, the aggregation of drug particles in the step forming drug particle can be remarkedly suppressed and additionally a sufficient charge amount can be obtained in the step preparing liquid coating agent, which is preferable.

In addition, when a cationic polymer is dissolved in the liquid coating agent, the surface of a biocompatible nanoparticle which is generally negative-charged can be also positive-charged together with a drug particle. Thereby, it becomes possible to electrically-attach the drug particle and the biocompatible nanoparticle to the body of a device electricity-conduced in the step attaching particle mentioned below, and then enhance the efficiency for attaching a particle. Additionally, the attached particle is tightly fixed, thus it is possible to prevent the removal of particle in the preparing process or in inserting and expanding the device.

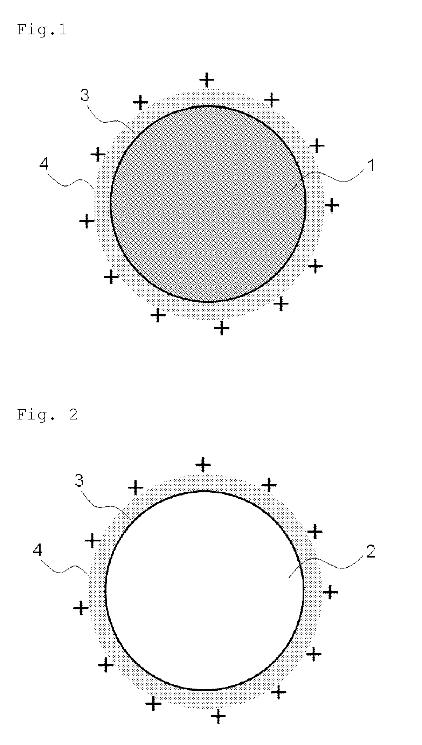

Fig. 1 and Fig. 2 show structures of a drug particle and a biocompatible nanoparticle which are dispersed in a liquid coating agent, respectively. Wherein, the surfaces of drug particle 1 and biocompatible nanoparticle 2 are coated with polyvinyl alcohol 3, and further the surfaces are coated with cationic polymer 4, in which cationic polymer 4 has positive zeta potential.

### (6) Step attaching particle

The next explanation is directed to a method for forming a particle layer by attaching a drug particle and a biocompatible nanoparticle to the body of a device. In the present invention, the formed particle layer is in a state that drug particles and biocompatible nanoparticles are mixed, wherein the drug particles have a relatively-broad particle size and the biocompatible nanoparticles have a small diameter. Thus, the interspace of the particles is small so that the close packing is possible. Consequently, the present invention can make a particle layer layered tightly, compared with the case of layering only drug particles, thereby the particle layer is a uniform one, and the amount of the coated drug particle is much. The process of attaching particle used herein includes a wiping method (coating the body of a device with a liquid coating agent, then drying it) and a dipping method (dipping the body of a device in a liquid coating agent, taking it out and drying it) .

In the step of the modification with positive-charge mentioned above, when the surfaces of drug particle and biocompatible nanoparticle are modified with positive-charge via dissolving a cationic polymer in a liquid coating agent, the process of attaching particle can be carried out by a method of electrically-attaching the drug particle and the biocompatible nanoparticle to the body of a device. Hereinafter, methods of attaching a particle to the body of a conductive stent, and methods of attaching a particle to the extensible attachment (balloon part) of a nonconductive catheter are explained.

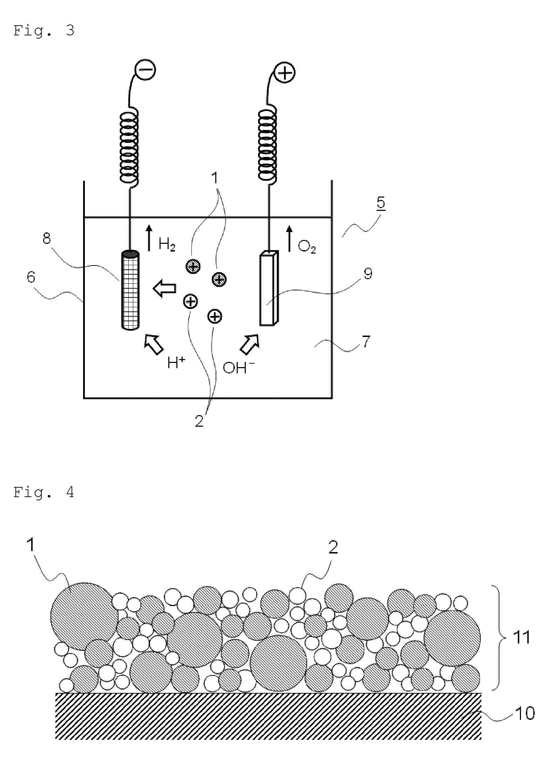

The method for preparing a DES in the present invention which attaches particles to the body of a conductive stent includes an electrophoresis wherein the body of a stent as a negative electrode is electrified in a suspension of drug particles and biocompatible nanoparticles, and a spraying method wherein a liquid drop containing drug particles and biocompatible nanoparticles is attached to the negative-charged surface of a stent. Fig 3 shows a diagrammatic illustration of the electrophoresis apparatus used for preparing a DES in the present invention. Electrophoresis apparatus 5 is filled with a suspension (liquid coating agent 7) of drug particle 1 and biocompatible nanoparticle 2 in its bath 6, which is composed of stent body 8 connected to the negative electrode side of an electrical circuit as a negative electrode and positive electrode 9 connected to the positive electrode side of an electrical circuit, both of which are dipped therein. In the illustration, stent body 8 used herein is made in a net-like cylindrical shape using a metallic fiber.

As mentioned above, drug particle 1 prepared in Step forming drug particle and Milling process and biocompatible nanoparticle 2 prepared in step forming nanoparticle are modified (coated) with a cationic polymer in Step of modification with positive-charge so that the zeta potential of the particle surface is positive-charged. Thus, when the electrical circuit is electrified in a state of Fig. 3, negative potential arises on the surface of stent body 8 so that positive-charged drug particle 1 and biocompatible nanoparticle 2 can attract to the surface to actively adhere to the surface. Water molecule in liquid coating agent 7 is also decomposed by an electric current and the liberated hydrogen ion (H⁺) is pulled to stent body 8 to get an electron from the surface of stent body 8, then hydrogen gas is generated. On the other hand, hydroxy ion (OH⁻) is pulled to positive electrode 9 to release an electron to positive electrode 9, then oxygen and water are generated.

With progression of the chemical reaction, a particle layer is formed, which is coated on the surface of stent body 8 in a state of mixed particles of drug particle 1 and biocompatible nanoparticle 2. And, the part of the formed particle layer is no longer conductive, thereby it is possible to form a uniform particle layer because forming the layer is not developed any more. In addition, it is easy to automatically proceed in the process of attaching particle, and it is possible to easily control the layer thickness by adjusting the electric voltage or the electrifying time, thereby the method is suitable for industrialization. Furthermore, in the invention, a coated object (the body of a stent) is used as a negative electrode, thus there is no dissolution of metal ion. Consequently, it is possible to attach the particle to iron, magnesium, etc.

Fig. 4 is a cross-sectional magnified drawing which shows a state in which particles are attached on a metallic fiber which is a component material of the body of a stent by electrophoresis. The surface of the negative-charged metallic fiber 10 is perfectly coated with the positive-charged drug particle 1 and biocompatible nanoparticle 2 to form a particle layer 11. Particle layer 11 formed by electrophoresis, which is densely layered in a mixed state of drug particle 1 and biocompatible nanoparticle 2, has a good uniformity, adhesion, and corrosion-resistance, compared with a particle layer which is prepared, for example, by dipping the body of a stent in a liquid coating agent and taking it out without electrifying the body of a stent.

Thereby, it is possible to prevent the peel-off of particle layer 11 from stent body 8 in the preparing process or in inserting a stent into a biologic body and expanding a stent. The cause of increasing the adhesion of particle layer 11 to stent body 8 by electrophoresis is van der Waals' force between the particles, etc.

The configuration of a stent body may be made by braiding a fiber material or cutting a metal pipe in a netlike appearance with laser, etc., which has a coronal shape, a roll shape, or a variety of conventional shapes known well. And, the stent body may be a balloon-extension type or a self-extension type, and the size of the stent body may be suitably chosen depending on the applied site. For example, in case of using in coronary artery, the preferred outer diameter before extended is generally about 1.0 to 3.0 mm, the preferred length is about 5.0 to 50 mm.

In attaching particles by electrophoresis, it is necessary to use a conductive material such as a metal for the stent body. The metal used for the stent body includes stainless, magnesium, tantalum, titanium, nickel-titanium alloy, inconel, gold, platinum, iridium, tungsten, and cobalt system alloy. In case that the stent is a self-extension type, the preferred metal is a hyperelastic alloy or the like such as nickel titanium because it is not necessary to re-form to the original shape. On the other hand, in case that the stent is a balloon-extension type, the preferred metal is stainless or the like which is hard to re-form to the original shape after the balloon is extended, in particular, SUS 316L which is the most corrosion-resistant is preferable.

The conductive material for the stent body besides a metal includes a conductive polymer such as polyaniline, polypyrrole, polythiophene, polyisothianaphthene, and polyethylenedioxythiophene, and conductive ceramics. In addition, it is acceptable in the present invention to use a nonconductive resin to which the conductiveness is given by adding a conductive filler or conductive-treating the surface with coating or the like.

When an un-biodegradable material such as stainless is used as a material of the stent body, it can cause restenosis because the stent is supposed to be placed for a long time and then can inflame the inner wall of a blood vessel. In that case, the patient has the necessity to be given PTCA (percutaneous transluminal coronary angioplasty) every a few months to re-place a stent, thus it was borne by the patient. To combat this, by using a biodegradable material as a material of the stent body, the inflammation caused by the stent placement can be suppressed because the stent body is gradually decomposed to disappear in a few months after the placement.

In using electrophoresis, the higher the electric voltage applied between the positive electrode and the negative electrode is, the more the amount of particles attracted to the surface of a stent in unit time is. Accordingly, the method has a merit that the formation of a particle layer on the surface of a stent can be carried out in a short time, but it is difficult to form a uniform particle layer because too many particles can be attached to the surface in one time. Thus, the electric voltage applied in electrophoresis may be suitably set considering the necessary uniformity of a particle layer and the efficiency forming a particle layer.

Next, the spray method is explained. The spray method is a method wherein a small suspension drop of positive-charged drug particle and biocompatible nanoparticle is attached to the surface of a stent body which is negative-charged by being electrified, which includes ultrasonic mist method which is carried out by making a mist of a liquid coating agent by ultrasonication, spray method wherein a liquid coating agent is sprayed to the surface of a stent by using a spray apparatus or an air brush, and air brush method.

In the spray method, the stent body is also negative-charged by being electrified. Thus, just like the case of electrophoresis, it is possible to prepare a DES which has a good adhesion between a stent body and a positive-charged particle and a good corrosion-resistance, compared with the spray method without electrifying the body of a stent. Additionally, in the spray method, it is possible to enhance the adhering efficiency of particles to the side or reverse face of a stent where is difficult to make a mist or a sprayed liquid drop thereof directly attached because a drug particle and a biocompatible nanoparticle in the liquid drop can actively adhere to the stent body. The configuration and quality of material of the stent body used in the spray method are as defined in case of electrophoresis.

When the particle layer formed on the surface of a stent is placed into a biologic body without any processing on the surface, the drug is released in a short time, thereby it is also difficult to control the releasing time. In order to obviate the problem, preferably, after forming a particle layer in the above process of attaching particle, the particle layer is impregnated with the solution of a biodegradable polymer before the particle layer is completely dried (impregnation process), then the particle layer is dried (dry process) to solidify the biodegradable polymer, and to form a biodegradable polymer layer.

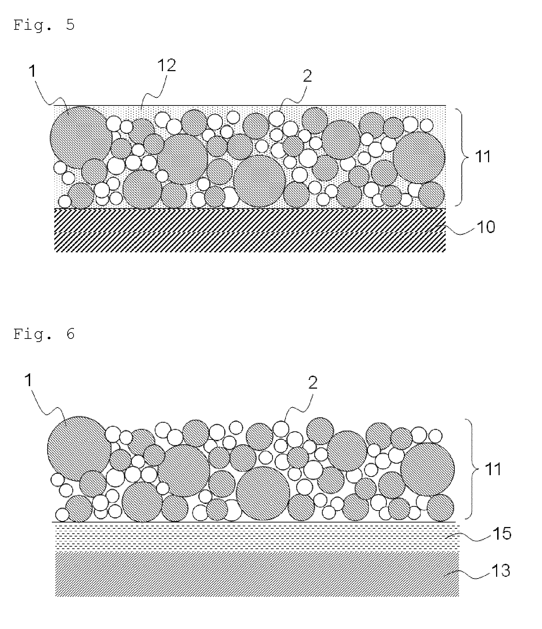

Fig. 5 shows a state in which a biodegradable polymer layer is formed through the impregnation process and dry process on the stent coated with a particle layer (Fig. 4). When particle layer 11 formed on the surface of metallic fiber 10 is impregnated with a solution of a biodegradable polymer before particle layer 11 is completely dried, the solution of a biodegradable polymer penetrates into the gap between drug particle 1 and biocompatible nanoparticle 2 which form particle layer 11. Then, by drying off the solvent which was used for dissolving a biodegradable polymer and the water remaining in particle layer 11, the gap in particle layer 11 can be filled with biodegradable polymer layer 12. Thus, each of drug particle 1 and biocompatible nanoparticle 2 can be kept there without agglutination thanks to the biodegradable polymer. Then, after placing a DES into a biologic body, drug particle 1 can be gradually eluted with the decomposition of biodegradable polymer layer 12, and then the drug can be taken into, for example, a vascular wall cell.

The biodegradable polymer used herein includes, for example, microbially-derived polymers such as polyhydroxybutyrate, and polyhydroxyvalerate; and naturally-occurring polymers such as collagen, acetylcellulose, bacterial cellulose, high-amylose cornstarch, starch, and chitosan. Amongst them, collagen and the like whose biologic decomposition rate is fast are more preferable than a biocompatible polymer used for forming a nanoparticle such as PLGA. It is possible to control the dissolution rate of drug particle 1 attached on the surface of a stent, through a suitable selection of the type of these biodegradable polymers, molecular weight thereof, and so on. It is possible to use PGA, PLA, PLGA, PAL and the like as a biodegradable polymer, then it is preferable to use a biodegradable polymer having a small molecular weight so that the decomposition rate thereof can be faster than that of biocompatible nanoparticle 2.

Next, a method of attaching a particle to the balloon part of a catheter is explained. The material of the balloon part includes a thermoplastic resin such as polyethylene, polypropylene, ethylene-propylene copolymer, ethylene-vinylacetate copolymer, bridged ethylene-propylene copolymer, bridged ethylene-vinylacetate copolymer, and polyvinyl chloride; and a nonconductive resin such as polyamide, polyurethane, polyester, and polyarylene sulfide. Accordingly, it is impossible to negative-charge the balloon part by being electrified.

Then, the balloon part can be modified with negative-charge beforehand, and a positive-charged drug particle and biocompatible nanoparticle can be electrically attached to the balloon part to make the particle tightly and uniformly attached to the balloon part. Preferably, the method for modifying a balloon part with negative-charge is a method to form negative-charged resin layer on the surface of the balloon part by using a negative-charged resin such as polycarboxylic acid and a polycarbonate derivative.

The polycarboxylic acid used for the modification with negative-charge includes a polymer of acrylic acid, methacrylic acid, maleic acid, fumaric acid, asparaginic acid or glutamic acid; a carboxymethyl derivative of starch, cellulose or polyvinyl alcohol; alginic acid; and pectin, and one or more kinds of them can be used.

In addition, the polycarbonate derivative used for the modification with negative-charge includes an acid anhydride or ester of the above-mentioned polycarboxylic acids. Amongst them, acid anhydrides or esters of polymers of acrylic acid, methacrylic acid, and maleic acid can make a low irritative and toxic modification with negative-charge for a biological body. The preferred polycarbonate derivative includes a copolymer of maleic anhydride such as maleic anhydride-methylvinyl ether copolymer, maleic anhydride-styrene copolymer, and maleic anhydride-ethylene copolymer, which are readily available and easy to handle. In particular, maleic anhydride-methylvinyl ether copolymer is preferable.

The method for coating a negative-charged resin layer used herein includes dipping the balloon part of a catheter in a solution of negative-charged resin; spraying a small liquid drop of a solution of negative-charged resin on the surface of the balloon part by ultrasonic mist method, spray method, air brush method and so on; and wiping the surface of the balloon part with a solution of negative-charged resin.

The method of attaching a particle to the balloon part used herein includes dipping the negative-charged-resin-layered balloon part of a catheter in a liquid coating agent; and attaching a liquid drop of a liquid coating agent to the balloon part by ultrasonic mist method, spray method, air brush method and so on.

Fig. 6 is a cross-sectional magnified drawing which shows a state in which particles are attached on the balloon part of a catheter. The surface of balloon part 13 is negative-charge-modified with negative-charged resin layer 15, the surface of negative-charged resin layer 15 is completely coated with positive-charged drug particle 1 and biocompatible nanoparticle 2, and then particle layer 11 is formed thereon.

Thereby, it is possible to prevent the peel-off of particle layer 11 from balloon part 13 in the preparing process or in inserting a catheter into a biologic body and expanding the balloon. The cause of increasing the adhesion of particle layer 11 to negative-charged resin layer 15 is van der Waals' force between the particles, etc.

The configuration of a catheter may be selected from a variety of conventional known shapes. And, the size of the catheter may be suitably chosen depending on the applied site. For example, in case of using in coronary artery, the preferred outer diameter before the balloon is extended is generally about 1.0 to 3.0 mm, the preferred length is about 5.0 to 50 mm.

Like the case in the process of attaching particles to a stent body, biodegradable polymer layer 12 may be formed via impregnation process and dry process in the process of attaching particle to balloon part 13 of a catheter.

In the medical device for placement into a lumen prepared herein, the cellular adhesiveness of the drug particle eluted from the surface of a device is increased because the surface of drug particle attached on the body of a device is positive-charged. Thereby, it is possible to enhance more the efficiency supporting a drug to stenotic cells in which a medical device should be placed than before.

In addition, two or more kinds of drug particles may be prepared, which may be attached in a layer state or a mosaic-like state. In this case, it is possible to systematically control each dissolution time of two or more kinds of drugs, for example, by attaching a drug particle which should be released in a short time after placing a device in a biologic body in the outer layer, and attaching a drug particle which should be released after a given time in the inner layer.

Furthermore, in case of forming a biodegradable polymer layer, when also adding a drug in a solution of a biodegradable polymer with which the particle layer is impregnated, each drug embedded in a drug particle and a biodegradable polymer layer can be acted simultaneously and rapidly. The type of drug to be added to a solution of the biodegradable polymer and the amount thereof can be suitably decided based on the action mechanism of the drug, a necessary quick effect, a necessary rate of substantivity, and so on.

For example, when it is necessary to make the efficacy of the drug sustained for a long time after the administration, the drug may be included inside a biocompatible nanoparticle or on the surface thereof, or when it is necessary to exert the efficacy of the drug from beginning soon after the administration, the drug may be included in a drug particle or in a biodegradable polymer layer. The drugs to be added into a biodegradable polymer layer include the various drugs exemplified in the biocompatible nanoparticle.

### EXAMPLES

The present invention should not be limited to the above-mentioned embodiments. The present invention can be varied and hence the technical scope of the present invention also includes embodiments obtained by connecting different technical means disclosed in different embodiments. Hereinafter, the present invention is in detail illustrated by the following examples using probucol or cilostazol as a poorly water-soluble drug and PLGA as a biocompatible polymer for forming a nanoparticle, about the preparations of drug particles (including probucol or cilostazol) whose surface is modified with positive-charge, the preparations of PLGA nanoparticles, and the preparations of a DES which is obtained by coating a device with them.

### Example 1 (Preparation of suspension containing drug particle, 1)

1 g of probucol was dissolved in a mixture of 40 mL of acetone and 20 mL of ethanol which are a good solvent for probucol to prepare a solution thereof. Separately, to 100 mL of 2 % (w/w) aqueous solution of polyvinyl alcohol (GOHSENOL EG05, NIPPON GOHSEI) was added 15 g of 2 % (w/w) aqueous solution of chitosan (CHITOSAN GH-400EF, NOF CORPORATION) to prepare a solution which is a poor solvent for probucol. To the solution at 40°C with stirring at 400 rpm, the afore-prepared solution of probucol was added dropwise in a constant speed (20 mL/min) to give a suspension of crystalline drug particle (probucol), which was prepared by so-called "diffusion of a good solvent into a poor solvent".
Consequently, the acetone and ethanol were removed in vacuo. The zeta potential on the particle surface was measured with an electrophoresis (Malvern Instruments, ZETASIZER Nano-Z) . The result showed that the zeta potential on the drug particle was +14 mV, which was a good dispersibility to water.
Consequently, the excess polyvinyl alcohol and chitosan were removed by centrifugal separation (20,000 rpm, 40 min). The precipitated drug particle was redispersed with water, and then the centrifugal separation was repeated.
35 g of the prepared suspension and 130 g of zirconia balls (φ 1 mm) were put into a 100 ml polystyrene cylindrical container and the container was sealed. The container was rolled on two rollers horizontally-aligned on a desktop-type ball mill (V-2M, IRIE SHOKAI) at 600 rpm for 2 hours to mill the drug particle. Then, the content in the container was filtrated with a filter to remove the zirconia balls and provide 1.2 % (w/w) suspension of drug particle.

The particle size of the drug particle in the solution was measured with a laser diffraction-scattering (NIKKISO, MICROTRAC MT3300). The content of probucol in the drug particle was evaluated with a high-performance liquid chromatograph (SHIMADZU, detector: SPD-20A, UV = 242 nm).
In the measurement of particle size with a laser diffraction-scattering, the mean particle size of drug particle was 2.9 µm, wherein agglomerated particles as well as simple particles are measured as a particle. In the following Examples and Comparative examples, each mean particle size of drug particles is measured with a laser diffraction-scattering unless otherwise indicated.
The content of probucol in the drug particle was 65 % and the yield ratio was 75 %.

### Example 2 (Preparation of suspension containing drug particle, 2)

A drug particle of Example 2 was prepared in the same manner as Example 1, provided that the drug particle was not milled with a desktop-type ball mill. The mean particle size of the drug particle was 21.5 µm.

### Example 3 (Preparation of suspension containing drug particle, 3)

A drug particle of Example 3 was prepared in the same manner as Example 1, provided that the amount of 2 % (w/w) aqueous solution of chitosan added to a solution equivalent to the poor solvent was 60 g. The mean particle size of the drug particle measured with a laser diffraction-scattering was 4.5 µm. The zeta potential was +45 mV, the content of probucol in the drug particle was 53 % and the yield ratio was 65 %.

### Comparative example 1 (Preparation of suspension containing drug particle, 4)

A drug particle of Comparative example 1 was prepared in the same manner as Example 1, provided that neither of polyvinyl alcohol nor chitosan was added to a solution equivalent to the poor solvent. The yield ratio was 1 %, which indicated that it was quite difficult to re-disperse the drug particle into water.

### Comparative example 2 (Preparation of suspension containing drug particle, 5)

A drug particle of Comparative example 2 was prepared in the same manner as Example 1, provided that polyvinyl alcohol was not added to a solution equivalent to the poor solvent. The yield ratio was 30 %, which indicated that it was difficult to re-disperse the drug particle into water.

### Comparative example 3 (Preparation of suspension containing drug particle, 6)

A drug particle of Comparative example 3 was prepared in the same manner as Example 1, provided that chitosan was not added to a solution equivalent to the poor solvent. The yield ratio was 25 %, which indicated that it was difficult to re-disperse the drug particle into water.

### Example 4 (Preparation of biocompatible nanoparticle)

2 g of a biocompatible polymer, poly(lactic-co-glycolic acid) (Wako Pure Chemical Industries, PLGA 7520, molecular weight 20,000, molar ratio of lactic acid / glycolic acid = 75/25) was dissolved in 320 mL of acetone which is a good solvent for the above poly(lactic-co-glycolic acid). 160 mL of ethanol was added thereto, and mixed to give a solution. Separately, 560 mL of 0.18 % (w/w) water solution of polyvinyl alcohol was prepared, which is a poor solvent for the above poly(lactic-co-glycolic acid). To the solution at 40°C with stirring at 400 rpm, the afore-prepared solution of poly(lactic-co-glycolic acid) was added dropwise in a constant speed (20 mL/min) to give a suspension of PLGA nanoparticle which is a biocompatible nanoparticle.

Consequently, the acetone and ethanol were removed in vacuo. The zeta potential on the particle surface was measured with an electrophoresis. The result showed that the zeta potential on the drug particle was -10 mV.
Then, the particle was lyophilized to give a powdered PLGA nanoparticle. The particle size of the PLGA nanoparticle was measured by dynamic light scattering. The mean particle size of the PLGA nanoparticle measured by dynamic light scattering was 0.28 µm.

### Example 5 (Preparation of liquid coating agent, 1)

To the suspension of drug particle prepared in Example 1 with stirring was added and dispersed the PLGA nanoparticle prepared in Example 4. Then, 2 % (w/w) aqueous solution of chitosan was added thereto, and the mixture was stirred for 1 hour to prepare a liquid coating agent. The mixture ratio of the drug particle : the PLGA nanoparticle : chitosan was 6 : 25 : 1 by weight.

### Example 6 (Preparation of liquid coating agent, 2)

A liquid coating agent of Example 6 was prepared in the same manner as Example 5, provided that the mixture ratio of the drug particle : the PLGA nanoparticle : chitosan was 6 : 12 : 1 by weight.

### Example 7 (Preparation of liquid coating agent, 3)

A liquid coating agent of Example 7 was prepared in the same manner as Example 5, provided that the mixture ratio of the drug particle : the PLGA nanoparticle : chitosan was 6 _{:} 3 _{:} 1 by weight.

### Example 8 (Preparation of liquid coating agent, 4)

A liquid coating agent of Example 8 was prepared in the same manner as Example 5, provided that the mixture ratio of the drug particle : the PLGA nanoparticle : chitosan was 6 : 1 : 1 by weight.

### Example 9 (Preparation of liquid coating agent, 5)

A liquid coating agent of Example 9 was prepared in the same manner as Example 5, provided that the suspension of drug particle used herein was the suspension of drug particle prepared in Example 2.

### Example 10 (Preparation of liquid coating agent, 6)

A liquid coating agent of Example 10 was prepared in the same manner as Example 5, provided that the suspension of drug particle used herein was the suspension of drug particle prepared in Example 3 and no aqueous solution of chitosan was added thereto.

### Comparative example 4 (Preparation of liquid coating agent, 7)

The suspension of drug particle prepared in Example 1 was stirred for 1 hour with a stirrer without adding the PLGA nanoparticle powder and 2 % (w/w) aqueous solution of chitosan, to prepare a liquid coating agent.

### Comparative example 5 (Preparation of liquid coating agent, 8)

To the suspension of drug particle prepared in Example 1 with stirring was added 2 % (w/w) aqueous solution of chitosan, and the mixture was stirred for 1 hour to prepare a liquid coating agent.

### Example 11 (Electrodeposition coating on the body of stent, 1)

The body of a stent made of stainless (SUS316L) (outer diameter: 2.3 mm, length: 16 mm) which was weighed beforehand was set at negative electrode, a circular SUS plate (diameter: 20 mm) was set at positive electrode, they were connected to an external power source, and then each of them (length: 10 mm) was dipped in the liquid coating agent prepared in Example 5 (n=3). They were electrified for one minute (electric voltage: 10 V, electric current 1 mA), then the body of the stent was taken off and air-dried to give a DES. After dried, the DES was weighed, and the mean weight of the solid layered on the body of the stent was calculated based on the increased weight.

### Example 12 (Electrodeposition coating on the body of stent, 2)

A DES of Example 12 was prepared in the same manner as Example 11, provided that the liquid coating agent prepared in Example 6 was used as the liquid coating agent (n=3). After dried, the DES was weighed, and the mean weight of the solid layered on the stent body was calculated based on the increased weight.

### Example 13 (Electrodeposition coating on the body of stent, 3)

A DES of Example 13 was prepared in the same manner as Example 11, provided that the liquid coating agent prepared in Example 7 was used as the liquid coating agent (n=3). After dried, the DES was weighed, and the mean weight of the solid layered on the stent body was calculated based on the increased weight.

### Example 14 (Electrodeposition coating on the body of stent, 4)

A DES of Example 14 was prepared in the same manner as Example 11, provided that the liquid coating agent prepared in Example 8 was used as the liquid coating agent (n=3). After dried, the DES was weighed, and the mean weight of the solid layered on the stent body was calculated based on the increased weight.

### Example 15 (Electrodeposition coating on the body of stent, 5)

A DES of Example 15 was prepared in the same manner as Example 11, provided that the liquid coating agent prepared in Example 9 was used as the liquid coating agent (n=3). After dried, the DES was weighed, and the mean weight of the solid layered on the stent body was calculated based on the increased weight.

### Example 16 (Electrodeposition coating on the body of stent, 6)

A DES of Example 16 was prepared in the same manner as Example 11, provided that the liquid coating agent prepared in Example 10 was used as the liquid coating agent (n=3). After dried, the DES was weighed, and the mean weight of the solid layered on the stent body was calculated based on the increased weight.

### Comparative example 6 (Electrodeposition coating on the body of stent, 7)

A DES of Comparative example 6 was prepared in the same manner as Example 11, provided that the liquid coating agent prepared in Comparative example 4 was used as the liquid coating agent (n=3). After dried, the DES was weighed, and the mean weight of the solid layered on the stent body was calculated based on the increased weight.

### Comparative example 7 (Electrodeposition coating on the body of stent, 8)

A DES of Comparative example 7 was prepared in the same manner as Example 11, provided that the liquid coating agent prepared in Comparative example 5 was used as the liquid coating agent (n=3). After dried, the DES was weighed, and the mean weight of the solid layered on the stent body was calculated based on the increased weight.

### Example 17 (Dip coating on the body of stent, 1)

A part (length: 10 mm) of the stent body made of stainless (SUS316L) (outer diameter: 2.3 mm, length: 16 mm) which was weighed beforehand was dipped in the liquid coating agent prepared in Example 5 (n=3). The body was kept to be dipped for one hour, and then it was dried in vacuo in a dry chamber (40°C, 3 hours). After dried, the DES was weighed, and the mean weight of the solid layered on the stent body was calculated based on the increased weight.

### Example 18 (Dip coating on the body of stent, 2)

A DES of Example 18 was prepared in the same manner as Example 17, provided that the liquid coating agent prepared in Example 9 was used as the liquid coating agent (n=3). After dried, the DES was weighed, and the mean weight of the solid layered on the stent body was calculated based on the increased weight.

### Example 19 (Dip coating on the body of stent, 3)

A DES of Example 19 was prepared in the same manner as Example 17, provided that the liquid coating agent prepared in Example 10 was used as the liquid coating agent (n=3). After dried, the DES was weighed, and the mean weight of the solid layered on the stent body was calculated based on the increased weight.

### Comparative example 8 (Dip coating on the body of stent, 4)

A DES of Comparative example 8 was prepared in the same manner as Example 17, provided that the liquid coating agent prepared in Comparative example 4 was used as the liquid coating agent (n=3). After dried, the DES was weighed, and the mean weight of the solid layered on the stent body was calculated based on the increased weight.

### Comparative example 9 (Dip coating on the body of stent, 5)

A DES of Comparative example 9 was prepared in the same manner as Example 17, provided that the liquid coating agent prepared in Comparative example 5 was used as the liquid coating agent (n=3). After dried, the DES was weighed, and the mean weight of the solid layered on the stent body was calculated based on the increased weight.

### Test 1 (Measurement of the amount of probucol attached on the stent body)

Each amount of probucol in the solid layered on the DESs prepared in Examples 11 to 19 and Comparative examples 6 to 9 was measured with a high-performance liquid chromatograph (n=3 in each group), and then each mean weight of the attached probucol was calculated. The results are shown in Tables 1 to 3 together with each mean weight of the solid layered on the stent body.

**Table 1**

| DES | Example 11 | Example 15 | Example 16 | Comparative example 6 | Comparative example 7 |
|---|---|---|---|---|---|
| Drug particle | Example 1 | Example 2 | Example 3 | Example 1 | Example 1 |
| PLGA nanoparticle | Added | Added | Added | Not added | Not added |
| Chitosan solution | Added | Added | Not added | Not added | Added |
| Weight of layered solid [mg] | 0.83 | 0.58 | 0.78 | 0.3 | 0.12 |
| Weight of attached probucol [mg] | 0.14 | 0.08 | 0.12 | 0.00 | 0.01 |

**Table 2**

| DES | Example 17 | Example 18 | Example 19 | Comparative example 8 | Comparative example 9 |
|---|---|---|---|---|---|
| Drug particle | Example 1 | Example 2 | Example 3 | Example 1 | Example 1 |
| PLGA nanoparticle | Added | Added | Added | Not added | Not added |
| Chitosan solution | Added | Added | Not added | Not added | Added |
| Weight of layered solid [mg] | 0.55 | 0.40 | 0.49 | 0.01 | 0.07 |
| Weight of attached probucol [mg] | 0.10 | 0.06 | 0.08 | 0.00 | 0.01 |

**Table 3**

| DES | Example 11 | Example 12 | Example 13 | Example 14 |
|---|---|---|---|---|
| (Drug particle: PLGA nanoparticle : chitosan) | (6 : 25 : 1) | (6 : 12 : 1) | (6 : 3 : 1) | (6 : 1 : 1) |
| Drug particle | Example 1 | Example 1 | Example 1 | Example 1 |
| PLGA nanoparticle | Example 4 | Example 4 | Example 4 | Example 4 |
| Chitosan solution | Added | Added | Added | Added |
| Weight of layered solid [mg] | 0.83 | 0.82 | 0.68 | 0.55 |
| Weight of attached probucol [mg] | 0.14 | 0.17 | 0.27 | 0.26 |

As it is clear in Table 1, in case of preparing a DES by electrodeposition-coating the stent body with the drug particle and the PLGA nanoparticle, it was observed that the amount of the probucol attached to the stent body was increased in the DESs of Examples 11 and 15 wherein PLGA nanoparticle and chitosan were added to the liquid coating agent. In the DES of Example 16 prepared by using the drug particle of Example 3 which was strongly positive-charged in forming a drug particle, it was observed that the amount of the probucol attached to the stent body was increased unless chitosan was used in preparing a liquid coating agent. To the DES of Example 11 prepared by using the drug particle of Example 1 wherein the primary particle size was micro-milled to about 2.9 µm with a ball mill, 0.14 mg of probucol was attached, which was about 1.8 times more than the amount of the attached probucol in Example 15 wherein the drug particle was not milled (the attached amount: 0.08 mg).
On the other hand, in the DES of Comparative example 6 wherein neither PLGA nanoparticle nor chitosan was added to the liquid coating agent, no or little layered solid was attached to the stent body. In case of Comparative example 7 wherein only chitosan was added, the amount of the attached probucol was 0.01 mg which was more than that of Comparative example 6, but the particle layer was not uniform and the amount of the attached probucol was much less than that of Example 11 or 15.

As it is clear in Table 2, also in case of preparing DESs by dip-coating the stent body with the drug particle and the PLGA nanoparticle, in the DESs of Examples 17 and 18 wherein PLGA nanoparticle and chitosan were added to the liquid coating agent, it was observed that probucol was attached thereto, compared with the DES of Comparative example 8 wherein neither PLGA nanoparticle nor chitosan was added and the DES of Comparative example 9 wherein only chitosan was added, though the amount of the attached probucol was little compared with the DESs of Examples 11, 15 and 16 prepared by electrodeposition coating. In the DES of Example 19 prepared by using the drug particle of Example 3 which was strongly positive-charged in forming a drug particle, it was observed that the amount of the probucol attached to the stent body was increased unless chitosan was used in preparing a liquid coating agent.
Furthermore, the difference of the amount of the attached probucol which arises depending on the presence or absence of micronization of the drug particle (comparing Examples 17 and 18), and the difference of the amount of the attached probucol which arises depending on the presence or absence of PLGA nanoparticle (comparing Comparative examples 8 and 9) tended the same as the case of the electrodeposition coating.
The above Examples show the experimental results of the case using probucol as a poorly water-soluble drug, but it is expected that devices comprising the other poorly water-soluble drugs also have the same effect as the case of probucol.

Table 3 summarizes the results of the amount of the attached probucol in the DESs prepared by electrodeposition coating, varying the mixing ratio of the drug particle, the PLGA nanoparticle and chitosan.
As increasing the content ratio of the PLGA nanoparticle per the drug, the weight of the solid layered on the stent was increased, but the attached probucol was decreased because the content ratio of probucol was lowered.
As increasing the content ratio of the PLGA nanoparticle, the particle layer formed on the stent body became hard to peel off. It is thought because the interspace of the particles is small to form a tight particle layer, as increasing the content ratio of the PLGA nanoparticle.

### Example 20 (Preparation of suspension containing cilostazol particle)

0.75 g of cilostazol was dissolved in a mixture of 108 mL of acetone and 60 mL of ethanol which are a good solvent for cilostazol to prepare a solution thereof. Separately, to 300 mL of 2 % (w/w) aqueous solution of polyvinyl alcohol (GOHSENOL EG05, NIPPON GOHSEI) was added 45 g of 2 % (w/w) aqueous solution of chitosan (CHITOSAN GH-400EF, NOF CORPORATION) to prepare a solution which is a poor solvent for cilostazol. To the solution at 40°C with stirring at 400 rpm, the afore-prepared solution of cilostazol was added dropwise in a constant speed (20 mL/min) to give a suspension of crystalline drug particle (cilostazol), which was prepared by so-called "diffusion of a good solvent into a poor solvent".
Consequently, the acetone and ethanol were removed in vacuo. The zeta potential on the particle surface was measured with an electrophoresis (Malvern Instruments, ZETASIZER Nano-Z). The result showed that the zeta potential on the drug particle was +7.4 mV, which was a good dispersibility to water.
Consequently, the excess polyvinyl alcohol and chitosan were removed by centrifugal separation (20,000 rpm, 40 min). The precipitated drug particle was redispersed with water, and then the centrifugal separation was repeated.
35 g of the prepared suspension and 130 g of zirconia balls (ϕ 1 mm) were put into a 100 ml polystyrene cylindrical container and the container was sealed. The container was rolled on two rollers horizontally-aligned on a desktop-type ball mill (V-2M, IRIE SHOKAI) at 600 rpm for 2 hours to mill the drug particle. Then, the content in the container was filtrated with a filter to remove the zirconia balls and provide 0.45 % (w/w) suspension of drug particle.

The particle size of the drug particle in the solution was measured with a laser diffraction-scattering (NIKKISO, MICROTRAC MT3300). The content of cilostazol in the drug particle was evaluated with a high-performance liquid chromatograph (SHIMADZU, detector: SPD-20A, UV = 254 nm).
In the measurement of particle size with a laser diffraction-scattering, the mean particle size of drug particle was 4.2 µm, wherein agglomerated particles as well as simple particles are measured as a particle. In the following Examples and Comparative examples, each mean particle size of drug particles is measured with a laser diffraction-scattering unless otherwise indicated.
The content of probucol in the drug particle was 98.6 % and the yield ratio was 82 %.

### Example 21 (Preparation of biocompatible nanoparticle containing cilostazol)

2 g of a biocompatible polymer, i.e. poly(lactic-co-glycolic acid) (Wako Pure Chemical Industries, PLGA 7520, molecular weight 20,000, molar ratio of lactic acid /glycolic acid = 75/25) and 10 mg of cilostazol were dissolved in 80 mL of acetone which are a good solvent. 40 mL of ethanol was added thereto, and mixed to give a solution. Separately, to 200 mL of 0.5 % (w/w) water solution of polyvinyl alcohol which is a poor solvent for the above poly(lactic-co-glycolic acid) was added 4.8 g of 2 % (w/w) aqueous solution of chitosan (CHITOSAN GH-400EF, NOF CORPORATION) and mixed to prepare a solution. To the solution at 40°C with stirring at 400 rpm, the afore-prepared solution of poly(lactic-co-glycolic acid) was added dropwise in a constant speed (20 mL/min) to give a suspension of PLGA nanoparticle comprising cilostazol which is a biocompatible nanoparticle.

Consequently, the acetone and ethanol were removed in vacuo. The zeta potential on the particle surface was measured with an electrophoresis. The result showed that the zeta potential was +46.7 mV.
Then, the particle was lyophilized to give a powdered PLGA nanoparticle. The particle size of the PLGA nanoparticle was measured by dynamic light scattering. The mean particle size of the PLGA nanoparticle measured by dynamic light scattering was 0.30 µm and the content of cilostazol in the biocompatible nanoparticle was 0.33 %.

### Example 22 (Preparation of liquid coating agent, 9)

To the suspension of drug particle prepared in Example 20 with stirring was added and dispersed the PLGA nanoparticle comprising cilostazol prepared in Example 21. Then, the mixture was stirred for 30 minutes to prepare a liquid coating agent. The mixture ratio of the drug particle : the PLGA nanoparticle was 1 _{:} 2 by weight.

### Example 23 (Preparation of liquid coating agent, 10)

A liquid coating agent of Example 23 was prepared in the same manner as Example 22, provided that the mixture ratio of the drug particle : the PLGA nanoparticle was 1 : 4.2 by weight.

### Comparative example 10 (Preparation of liquid coating agent, 11)

The suspension of drug particle prepared in Example 20 was stirred for 30 minutes with a stirrer without adding the PLGA nanoparticle comprising cilostazol prepared in Example 21, to prepare a liquid coating agent.

### Comparative example 11 (Preparation of liquid coating agent, 12)

To the PLGA nanoparticle comprising cilostazol prepared in Example 21 was added purified water so that the concentration of the PLGA nanoparticle should be the same as Examples 22 and 23, and the mixture was stirred for 30 minutes with a stirrer without adding the drug particle prepared in Example 20, to prepare a liquid coating agent.

### Example 24 (Electrodeposition coating on the body of stent, 9)

The stent body made of stainless (SUS316L) (outer diameter: 2.3 mm, length: 16 mm) which was weighed beforehand was set at negative electrode, a circular SUS plate (diameter: 20 mm) was set at positive electrode, they were connected to an external power source, and then each of them (length: 10 mm) was dipped in the liquid coating agent prepared in Example 22 (n=3). They were electrified for one minute (electric voltage: 10 V, electric current 1 mA), then the stent body was taken off and air-dried to give a DES. After dried, the DES was weighed, and the mean weight of the solid layered on the stent body was calculated based on the increased weight.

### Example 25 (Electrodeposition coating on the body of stent, 10)

A DES of Example 25 was prepared in the same manner as Example 24, provided that the liquid coating agent prepared in Example 23 was used as the liquid coating agent (n=3). After dried, the DES was weighed, and the mean weight of the solid layered on the stent body was calculated based on the increased weight.

### Comparative example 12 (Electrodeposition coating on the body of stent, 11)

A DES of Comparative example 12 was prepared in the same manner as Example 24, provided that the liquid coating agent prepared in Comparative example 10 was used as the liquid coating agent (n=3). After dried, the DES was weighed, and the mean weight of the solid layered on the stent body was calculated based on the increased weight.

### Comparative example 13 (Electrodeposition coating on the body of stent, 12)

A DES of Comparative example 13 was prepared in the same manner as Example 24, provided that the liquid coating agent prepared in Comparative example 11 was used as the liquid coating agent (n=3). After dried, the DES was weighed, and the mean weight of the solid layered on the stent body was calculated based on the increased weight.

### Test 2 (Measurement of the amount of cilostazol attached on the stent body)

Each amount of cilostazol in the solid layered on the DESs prepared in Examples 24 and 25, and Comparative examples 12 and 13 was measured with a high-performance liquid chromatograph (n=3 in each group), and then each mean weight of the attached cilostazol was calculated. The results are shown in Table 4 together with each mean weight of the solid layered on the stent body and the rate of cilostazol in layered solid.

**Table 4**

| DES | Example 24 | Example 25 | Comparative example 12 | Comparative example 13 |
|---|---|---|---|---|
| Drug particle | positive-charge | positive-charge | positive-charge | - |
| PLGA nanoparticle | positive-charge | positive-charge | - | positive-charge |
| Weight of layered solid [mg] | 0.827 | 0.813 | Hard to measure | 0.776 |
| Rate of cilostazol in layered solid [%] | 40.6 | 22.4 | Hard to calculate | 0.33 |
| Weight of attached cilostazol [mg] | 0.336 | 0.182 | 0.011 | 0.003 |

As it is clear in Table 4, in case of preparing DESs by electrodeposition-coating the stent body with the drug particle and the PLGA nanoparticle, it was observed that the amount of the cilostazol attached to the stent body was increased in the DESs of Examples 24 and 25 wherein PLGA nanoparticle was added to the liquid coating agent.
On the other hand, in the DES of Comparative example 12 wherein no PLGA nanoparticle was added to the liquid coating agent, the layered solid was attached to the stent body, but the particle layer was not uniform.
In case of the DES of Comparative example 4 wherein no drug particle was added to the liquid coating agent, the amount of the layered solid was more than that of Comparative example 12, but the amount of the cilostazol attached to the stent was 0.01 mg or less which was much less than that of Example 24 or 25, because the content of cilostazol in the PLGA nanoparticle was low.

### Test 3 (In vivo test using stent coated with drug)

The stent of Example 24 on which cilostazol was attached was applied to a miniature swine, and the damage of the applied blood vessel was evaluated.

### Animal species

Miniature swine (line: NIBS, NISSEIKEN CO.LTD)
   male: 8, female: 4
   month old: 10 to 16 months old (when obtained)
   body weight: 20 to 28 kg (when obtained)

### Test group

### Cilostazol-attached stent group: n=12

(The stent system used herein was the stent of Example 24.)

### Control group: n=12

(The stent system used herein was a non-coated stent corresponding to the above.)

### Operation method

The swine were anesthetized with ketamine hydrochloride (500 to 750 mg/miniature swine, intramuscular injection, Ketalar^{®} 50, Sankyo Yell Pharmaceutical Co.Ltd). A guidewire (Radifocus Guidewire M, TERUMO CORPORATION) and a guiding catheter (Heartrail II, AMPLATZ LEFT Short Tip, TERUMO CORPORATION) were inserted into the blood vessel, and they were moved to the heart. The stent system of each test group was inserted into the blood vessel along the guidewire, and they were moved to the left anterior descending artery or the left circumflex artery where is to be placed. After inflation for 30 seconds, the stent was placed. After the stent was placed, Nitorol was administered, and the coronary artery was imaged. The catheter was taken off, and the incision site was disinfected (with Isodin^{Ⓡ} liquid for animal, Meiji Co., Ltd.), the muscle and skin were sutured. For each one miniature swine, both one cilostazol-attached stent and one control stent were applied.

### Angiography

The blood vessel of the site where the stent was placed was imaged. A catheter was inserted from the femoral artery, the tip of the catheter was moved to the aperture of the coronary artery, a contrast agent was injected from the catheter, and the angiographic photographs of the site where the stent was placed were taken. The photographing was carried out three times in total, i.e., before the placement of stent, after the placement of stent, and at the anatomy.

### Concomitant drug, dose, administration method and dosing period

### 100 mg of aspirin once/day (administered with food)

Dosing period: two days before the operation to the end of the experiment.

### 50 mg of clopidogrel sulfate once/day (administered with food)

Dosing period: two days before the operation to two weeks after the operation including the operation day. Antibiotic 100 mg/ kg, once/day (intramuscular injection)
Dosing period: for three days after the operation including the operation day.
Note: Isosorbide dinitrate (Nitorol), lidocaine (Xylocaine), noradrenaline, atropine sulfate and so on are prepared for spasm, arrhythmia, hypotension and the like which are predicted to happen after the operation.

### Extirpation the blood vessel where stent is placed

28 days after the stent was placed, the groin of the miniature swine was opened under anesthesia, an angiographic catheter was inserted, and then the coronary artery was imaged, in the same manner at the placement. After the imaging, the animal was sacrificed by bleeding, and the coronary artery where the stent was placed was taken out. The extirpated blood vessel was fixed in 10 % neutral buffered formalin, and it was sent to Histo Science Laboratory Co., Ltd. At three sites, i.e. at about 1.5 mm of the stent origin side, at the center site and at about 1.5 mm of the terminal of the stent, each stent sample was prepared to HE-stained.

### Result

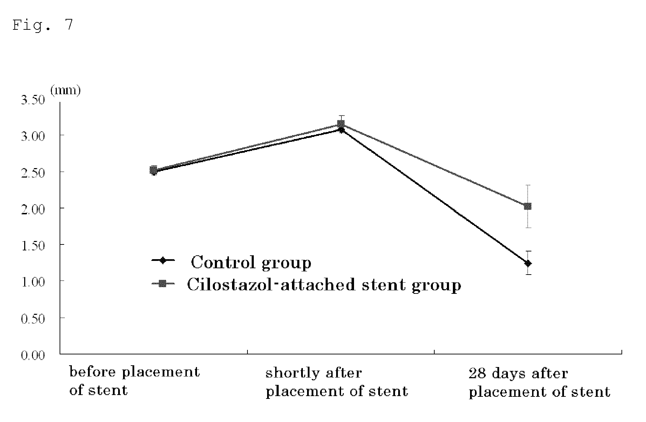

With the angiographic photographs, the minimum vessel diameter was measured before the placement of the stent, shortly after the placement of the stent and at the anatomy, and the transitional result is shown in Fig. 7. The coarctation of the vessel diameter in the cilostazol-attached stent group was significantly suppressed, compared with the control group.

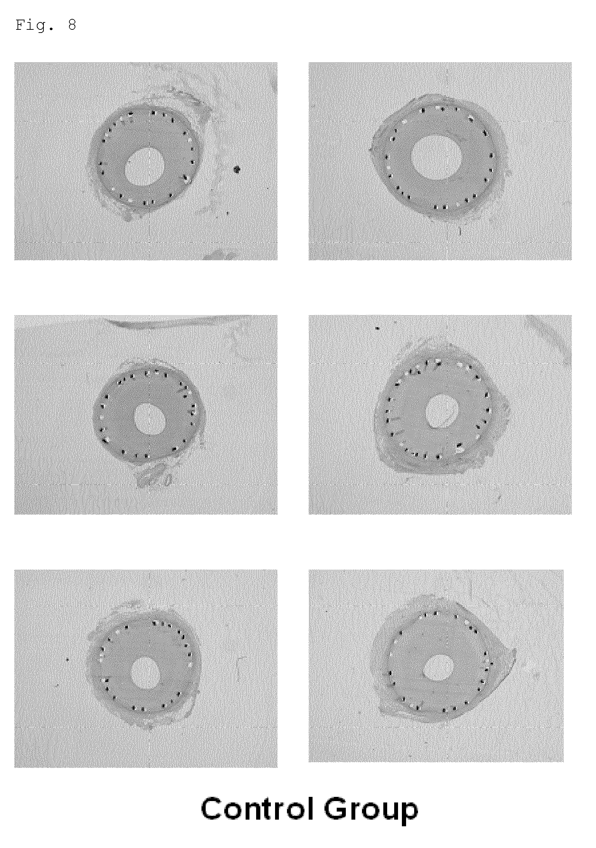

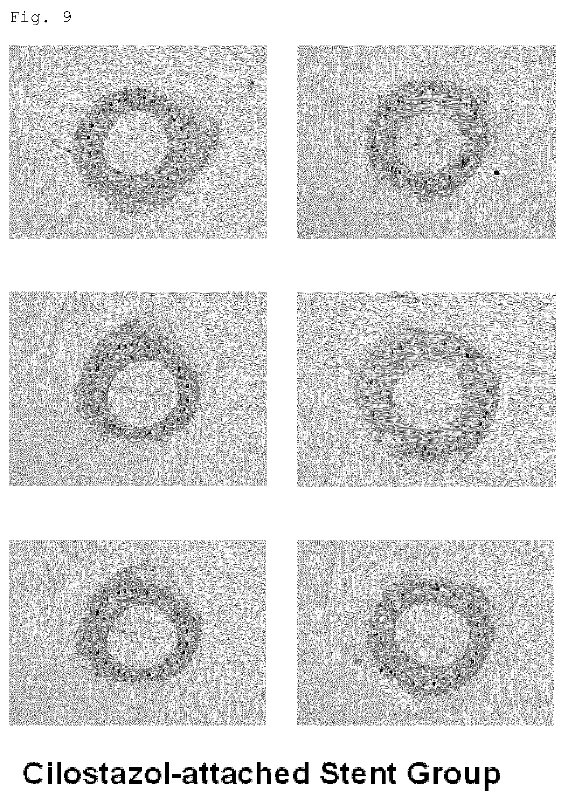

Fig. 8 shows the result in the control group of HE-stained pathological specimens of the cross-sectional vessel which was extirpated 28 days after the placement of the stent, and Fig. 9 shows the result of the cilostazol-attached stent group.
In the results of Fig. 8 and Fig. 9, the figures are, from above, as to the stent origin side, the center site, and the terminal of the stent, wherein each two figures of left and right are derived randomly from the miniature swine. The stenosis in the blood vessel in the cilostazol-attached stent group was more inhibited compared with the control group.

### INDUSTRIAL APPLICABILITY

The medical device for placement into a lumen of the present invention is a device wherein the body of the device is coated with mixed particles of a drug particle whose surface is positive-charged and a biocompatible nanoparticle, thereby it is possible to make the particle layer uniformed to coat the device uniformly with a sufficient amount of the drug. Additionally, in the present invention, the drug particle is dissolved in a short time after placing a medical device in a biological body, thereby it is expected to exert a rapid action of the drug, and the cellular adhesiveness of the drug particle dissolved in a biological body can be increased thanks to the modification of positive-charge and the transitivity into a cell can be also increased.

In addition, according to the method for preparing a medical device for placement into a lumen of the present invention in which the device body is coated with a drug particle whose surface is modified with positive-charge and a biocompatible nanoparticle, it is possible to prepare a medical device for placement into a lumen in which the surface of the device body is effectively and uniformly coated with a poorly water-soluble drug which was difficult to be attached to the device body until now, by easy way and with low cost. The process of attaching particle used herein includes preferably electrophoresis, ultrasonic mist method, spray method, air brush method, wiping method and dipping method.

## Claims

1. A medical device for placement into a lumen whose body is coated with mixed particles of poorly water-soluble drug particles whose surface is modified with positive-charge and biocompatible nanoparticles.

2. The medical device for placement into a lumen of claim 1 wherein the surface of the drug particles is modified with positive-charge by attaching a cationic polymer to their surface.

3. The medical device for placement into a lumen of claim 2 wherein the cationic polymer is chitosan or a chitosan derivative.

4. The medical device for placement into a lumen of any one of claims 1 to 3 wherein the drug particles have a mean particle size of 0.1 µm to 5 µm.

5. The medical device for placement into a lumen of any one of claims 1 to 4 wherein the biocompatible nanoparticles have a mean particle size of 300 nm or less.

6. The medical device for placement into a lumen of any one of claims 1 to 5 wherein the biocompatible nanoparticle supports the same or different drug inside and/or on its surface besides the drug particle.

7. The medical device for placement into a lumen of any one of claims 1 to 6 wherein the biocompatible nanoparticle is composed of any one of polylactic acid, polyglycolic acid, poly(lactic-co-glycolic acid), and lactate-aspartate copolymer.

8. The medical device for placement into a lumen of any one of claims 1 to 7 which is a drug-dissolution type stent whose body is coated with the drug particles and the biocompatible nanoparticles.

9. The medical device for placement into a lumen of any one of claims 1 to 7 which is a drug-dissolution type catheter coated with the drug particles and the biocompatible nanoparticles.

10. The medical device for placement into a lumen of any one of claims 1 to 7 which is a drug-dissolution type balloon catheter coated with the drug particles and the biocompatible nanoparticles.

11. The medical device for placement into a lumen of any one of claims 1 to 10 wherein the drug of the drug particles is cilostazol.

12. The medical device for placement into a lumen of any one of claims 1 to 11 which is for preventing and/or treating thrombus, stenosis, and/or restenosis.

13. A method for preventing and/or treating thrombus, stenosis, and/or restenosis which comprises using the medical device for placement into a lumen of any one of claims 1 to 11.

14. Use of the medical device for placement into a lumen of any one of claims 1 to 11 for preventing and/or treating thrombus, stenosis, and/or restenosis.

15. A liquid coating agent for coating a medical device for placement into a lumen which is a suspension prepared by dispersing drug particles modified with positive-charge by attaching a cationic polymer to their surface and biocompatible nanoparticles in a water solution.

16. The liquid coating agent of claim 15 wherein the drug particles have a mean particle size of 0.1 µm to 5 µm, the biocompatible nanoparticles have a mean particle size of 300 nm or less, and the biocompatible nanoparticle is composed of any one of polylactic acid, polyglycolic acid, poly(lactic-co-glycolic acid), and lactate-aspartate copolymer.

17. The liquid coating agent of claim 15 or 16 wherein the drug of the drug particles is cilostazol.

18. The liquid coating agent of any one of claims 15 to 17 wherein the medical device for placement into a lumen is used for preventing and/or treating thrombus, stenosis, and/or restenosis.

19. A process for preparing a medical device for placement into a lumen which comprises
(1) a step forming a drug particle by adding a solution of a drug in an organic solvent to an aqueous solution of a water-soluble polymer to provide a drug particle,
(2) a step forming a nanoparticle by adding a solution of a biocompatible polymer in an organic solvent to an aqueous solution of a water-soluble polymer to provide a biocompatible nanoparticle,
(3) a step preparing a liquid coating agent by dispersing the above-formed drug particle and the above-formed biocompatible nanoparticle to provide a liquid coating agent,
(4) a step modifying the surface of the drug particle with positive-charge by dissolving a cationic polymer in the aqueous solution in step (1) and/or the liquid coating agent in step (3), and
(5) a step coating the device with the above-prepared liquid coating agent by contacting the liquid coating agent and the body of the device to form a particle layer in a state of mixed particles of the drug particles and the biocompatible nanoparticles.

20. The process of claim 19 wherein in step (4) the surface of the drug particle is modified with positive-charge by dissolving a cationic polymer in both the aqueous solution in step (1) and the liquid coating agent in step (3).

21. The process of claim 19 or 20 which further comprises a step milling the drug particles after step (1).

22. The process of any one of claims 19 to 21 wherein in step (1) the water-soluble polymer is polyvinyl alcohol and the cationic polymer is chitosan.

23. The process of any one of claims 19 to 22 wherein step (5) is carried out by electrophoresis, ultrasonic mist method, spray method, air brush method, wiping method or dipping method.

24. The process of any one of claims 19 to 23 wherein the drug particles are composed of two or more kinds of drug particles which are in a layered state or in a mosaic state.

25. The process of any one of claims 19 to 24 wherein the drug of the drug particles is cilostazol.
